# EUROPEAN PATENT APPLICATION

(11) **EP 4 763 081 A1**
(43) Date of publication of application: **24.06.2026**
(21) Application number: 25226502.0
(22) Date of filing: 22.12.2025
(51) Int. Cl.: A61B 5/15, A61B 5/151

(54) **BLOOD EXTRACTION DEVICE**

(30) Priority: 20.12.2024 IN 202441101423
(71) Applicant: Remidio Innovative Solutions Private Limited, Bangalore 560058 (IN)
(72) Inventor: KOTTAYA, Pramod Kummaya, 5606062 BANGALORE (IN); C P, Aswin, 673102 KOZHIKODE (IN)
(74) Representative: Regimbeau

(57) **Abstract**

The present subject matter discloses a blood extraction device 100 comprising a main body 102 defining an internal cavity 102a including a vacuum chamber 104 and a lancet assembly 106. The lancet assembly 106 includes a lancet spring 106a and a needle 106b aligned with a skin-contact opening formed at a lower surface of the main body 102. An adhesive layer 110 is disposed on the lower surface of the main body 102 and is configured to attach the device to a user's skin such that the region is sealed around the skin-contact opening. An actuator knob 112 is mounted on a top surface 102b of the main body 102 and is mechanically coupled to a rotating valve 114b positioned within the internal cavity 102a. Rotation of the actuator knob 112 actuates the lancet spring to drive the needle toward the skin-contact opening and applies vacuum for blood extraction.

## Description

### BACKGROUND

Blood collection is a fundamental procedure in medical diagnostics, health monitoring, and disease prevention. It is often necessary to obtain a blood sample from a patient for analysis of various diseases. For instance, in the case of a specific infectious disease, a blood sample can be analyzed for the presence of blood-borne pathogens. Similarly, in diabetes, blood glucose levels can be measured using periodically collected blood samples. There are multiple techniques for obtaining blood samples. The most common technique is venipuncture, where a needle is inserted into a vein to collect blood. Another technique is capillary sampling using fingerstick sampling, which involves a small prick on the fingertip. Each of these techniques has its own advantages and disadvantages.

With the advancement in technology in the medical field, various strategies have been proposed to ensure that the blood extraction is safe, reliable and accessible.

### BRIEF DESCRIPTION OF FIGURES

The detailed description is described with reference to the accompanying figures. In the figures, the left-most digit(s) of a reference number identifies the figure in which the reference number first appears. The same numbers are used throughout the drawings to reference features and components.
Figure 1 illustrates a perspective view of the blood extraction device, in accordance with an example implementation of the present subject matter;
Figure 2A illustrates a cross-sectional view of the blood extraction device, in accordance with an example implementation of the present subject matter;
Figure 2B illustrates another cross-sectional view of the blood extraction device, in accordance with an example implementation of the present subject matter;
Figure 2C illustrates a cross-sectional view of the blood extraction device, in accordance with another example implementation of the present subject matter;
Figure 3 illustrates an exploded view of the blood extraction device, in accordance with an example implementation of the present subject matter;
Figure 4A illustrates a perspective view of the blood extraction devices in a loaded state, in accordance with an example implementation of the present subject matter;
Figure 4B illustrates a cross-sectional view of the blood extraction devices in the loaded state, in accordance with an example implementation of the present subject matter;
Figure 4C illustrates another perspective view of the blood extraction devices in an unloaded state, in accordance with an example implementation of the present subject matter;
Figure 4D illustrates another cross-sectional view of the blood extraction devices in the unloaded state, in accordance with an example implementation of the present subject matter;
Figures 5A-5B illustrate perspective views of a base plate of the blood extraction device and an associated support part, in accordance with an example implementation of the present subject matter;
Figures 5C-5D illustrate perspective views of a top plate of the blood extraction device and an associated support part, in accordance with an example implementation of the present subject matter;
Figure 6A illustrates a top view of the blood extraction device in the loaded state, in accordance with another example of a present subject matter;
Figure 6B illustrates the top view of the blood extraction device in the unloaded state, in accordance with another example of a present subject matter;
Figure 7 illustrates the top view of the blood extraction device having a vacuum indicator, in accordance with another example of a present subject matter;
Figure 8A illustrates a front view of the blood extraction device, in accordance with another example of a present subject matter; and
Figure 8B illustrates a front view of the blood extraction device, in accordance with another example of a present subject matter;

It may be noted that throughout the drawings, identical reference numbers designate similar, but not necessarily identical, elements. The figures are not necessarily to scale, and the size of some parts may be exaggerated to more clearly illustrate the example shown. Moreover, the drawings provide examples and/or implementations consistent with the description; however, the description is not limited to the examples and/or implementations provided in the drawings.

### DETAILED DESCRIPTION

Traditional blood sampling techniques, such as venipuncture and fingerstick lancets present several challenges for both healthcare providers and patients. For instance, venipuncture requires trained medical professionals, sterile environment, and access to veins, which may not be feasible in remote or at-home settings. It can also be uncomfortable and anxiety inducing for patients, especially children and the elderly. On the other hand, fingerstick devices, while more accessible, often cause significant pain due to deeper penetration of the skin and typically involves a limited amount of blood. The blood collection process can be inconsistent, requiring manual squeezing of the finger, which affects the quality of blood sample.

Another significant problem with the conventional blood collection devices is the risk of blood contamination and clotting. Traditional lancets require manual handling of blood drops, often transferring blood from skin to test strip or collection tube, which increases the risk of errors and contamination. Clotting can also occur when the blood is exposed to air, leading to unusable samples. This issue becomes more pronounced when dealing with small blood volumes needed for microfluidics or lab-on-chip diagnostic devices. Ensuring proper sample collection, avoiding clotting, and minimizing user intervention are critical challenges that current solutions do not fully address.

Generally, the current blood collection devices in the market require specific technical skills, access to sterile environments, or the availability of healthcare professionals, making them unsuitable for use in non-clinical settings. This limits access to essential blood testing for patients in remote or resource limited areas. Self-testing kits like glucometer use fingerstick method, but they are often painful, require active user intervention, and provide insufficient blood for more advanced diagnostic tests. Additionally, these devices are not always user-friendly or elderly-friendly, making them less inclusive. Therefore, there is a need for a simple, user-friendly device that enables blood extraction with minimal discomfort and no technical expertise.

To address the challenges posed by current blood extraction devices, a novel design for a device for extracting blood is described herein that overcomes the limitations of conventional blood collection devices.

The present subject matter discloses a blood extraction device. The device comprises a main body defining an internal cavity. The internal cavity includes a vacuum chamber and a lancet assembly, the lancet assembly having a lancet spring and a needle coupled to the lancet spring. The needle is aligned with a skin-contact opening formed at a lower surface of the main body. An adhesive layer is disposed on the lower surface and is configured to attach the main body to a region of a user's skin, thereby sealing the region around the skin-contact opening. An actuator knob is mounted on a top surface of the main body. A rotating valve is positioned within the internal cavity and coupled to the lancet assembly, the rotating valve comprising a valve port configured to align with a fluid passage extending between the vacuum chamber and the skin-contact opening in the main body. The actuator knob is mechanically coupled to both the rotating valve and to the lancet spring such that rotation of the actuator knob actuates the lancet spring to move the needle toward the skin-contact opening to pierce the user's skin and simultaneously rotates the rotating valve to align the valve port with the fluid passage. This alignment establishes fluid communication between the vacuum chamber and the skin-contact opening, allowing blood to be drawn from the user's skin under the negative pressure of the vacuum chamber.

The present subject matter provides a significant improvement over the existing blood extraction devices by integrating a vacuum-assisted capillary blood collection system with a safe, automated lancet mechanism. The combination of simultaneous skin puncture and blood collection using vacuum reduces procedural complexity, making it suitable for home use, point-of-care testing, and remote areas. Additionally, by incorporating an adhesive layer, the need for manual pressure or external support to maintain device stability during blood collection is eliminated. This adhesive-based attachment not only ensures consistent vacuum pressure but also enhances user comfort by reducing the risk of slippage or misalignment.

The above-mentioned implementations are further described herein with reference to the accompanying figures. It should be noted that the description and figures relate to exemplary implementations and should not be construed as a limitation to the present subject matter. It is also to be understood that various arrangements may be devised that, although not explicitly described or shown herein, embody the principles of the present subject matter. Moreover, all statements herein reciting principles, aspects, and embodiments of the present subject matter, as well as specific examples, are intended to encompass equivalents thereof.

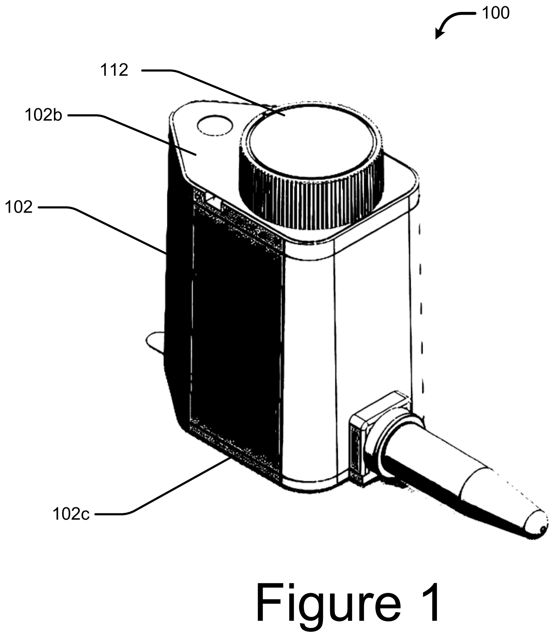

For further elaboration, reference is made to Figure 1, which is illustrating perspective view of the blood extraction device in accordance with an example implementation of the present subject matter. The blood extraction device 100 comprises a compact and self-contained configuration suitable for single-use capillary blood collection. The blood extraction device 100 is designed to be simple, user-friendly, and safe for home or clinical use.

In an example, the blood extraction device 100 includes a main body 102 defining an internal volume or cavity 102a configured to accommodate functional components associated with lancet actuation, vacuum generation, and blood collection. In an example, the main body 102 may be formed as a substantially hollow housing having an overall geometry adapted to be comfortably handled and positioned on a user's skin.

In an example, the main body 102 includes a top portion 102b and a bottom portion 102c. The top portion 102b is configured to support user-accessible components and may provide structural mounting for an actuation interface. In the illustrated example, an actuator knob 112 may be disposed on the top portion 102b of the main body 102. The actuator knob 112 is configured to be manually rotated by a user to initiate operation of the blood extraction device 100, as described in further detail herein.

In other examples, the actuation interface may comprise alternative user-operable mechanisms, including but not limited to a push button, slider, lever, press-to-release member, or any other mechanical, electromechanical, or hybrid actuation arrangement capable of triggering the internal operating components of the blood extraction device 100.

The bottom portion 102c of the main body 102 is configured to face and engage a skin surface of a user during use. In some examples, the bottom portion 102c of the blood extraction device 100 may comprise an adhesive arrangement (not shown in this figure) configured to attach the blood extraction device 100 to a target skin region and to form a substantially leak-proof seal around a skin-contact opening. Such a configuration facilitates stable positioning of the blood extraction device 100 and enables controlled application of negative pressure during blood extraction.

In operation, the blood extraction device 100 is positioned on a user's skin, for example on an upper arm region, after removal of a protective layer covering the adhesive arrangement provided at the bottom portion 102c. Upon placement, the adhesive arrangement secures the main body 102 to the skin, thereby maintaining positional stability and sealing the interface between the device and the skin. Once attached, the user actuates the blood extraction device 100 by rotating the actuator knob 112 from an initial position to an actuated position. Rotation of the actuator knob 112 causes internal mechanical components housed within the main body 102 to operate in a coordinated manner, including actuation of a lancet assembly and establishment of fluid communication between a vacuum source and the skin-contact opening. As a result, capillary blood is extracted from the skin and directed into a blood collection arrangement coupled to the blood extraction device 100.

The detailed configuration and operation of internal components housed within the main body 102, including but not limited to the lancet assembly, vacuum chamber, valve arrangement, blood flow passages, and associated actuation and control mechanisms, are described subsequently.

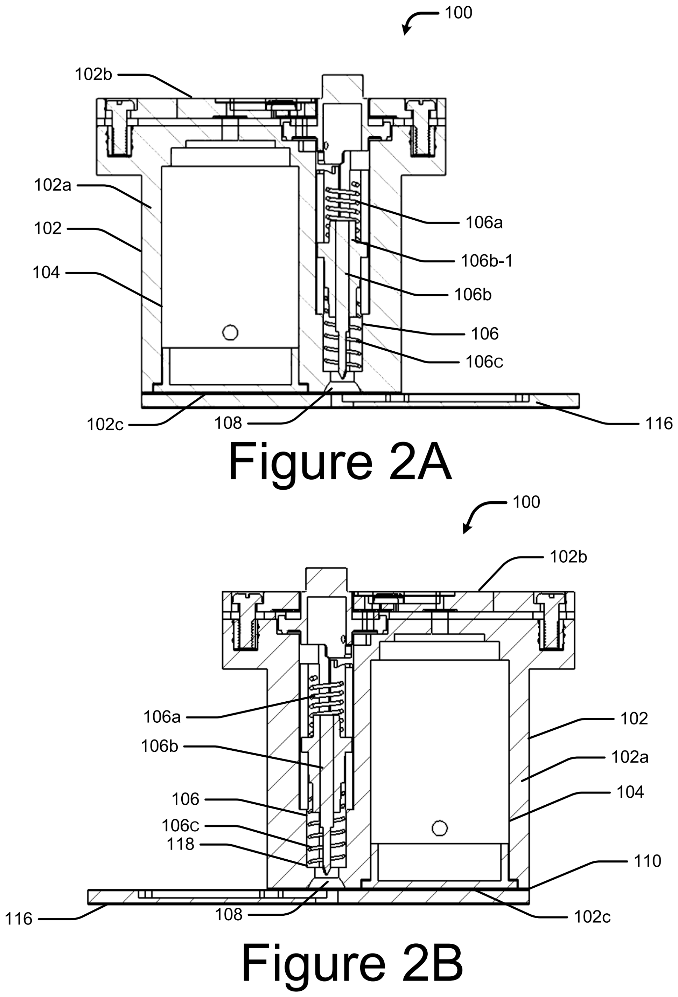

Figures 2A-2B illustrate cross-sectional views of the blood extraction device 100 in accordance with example implementations of the present subject matter. For the sake of brevity, Figures 2A-2B are described together herein.

In an example, the blood extraction device 100 comprises a main body 102 defining an internal cavity 102a. The internal cavity 102a is configured to house functional components associated with lancet actuation, vacuum application, and blood collection. The main body 102 includes a top portion 102b and a bottom portion 102c, wherein the bottom portion 102c is configured to face and engage a skin surface of a user during use. An actuator knob 112 is mounted on the top portion 102b of the main body 102. The actuator knob 112 is mechanically coupled to internal components disposed within the internal cavity 102a, such that manual actuation of the actuator knob 112 initiates coordinated operation of the blood extraction device 100, including actuation of a lancet assembly and application of negative pressure.

The main body 102 of the blood extraction device 100 comprises a vacuum chamber 104 disposed within the internal cavity 102a. The vacuum chamber 104 is configured to contain negative pressure prior to actuation and to selectively apply such negative pressure to a skin-contact opening during operation of the device. The vacuum chamber 104 is positioned within the main body 102 so as to be fluidly connectable to the skin-contact opening 108 through an internal fluid passage when actuated.

In an example, the main body 102 of the blood extraction device 100 further comprises a lancet assembly 106 disposed within internal cavity 102a. The lancet assembly 106 comprises a lancet spring 106a and a needle 106b coupled to the lancet spring 106a, wherein the needle 106b is aligned with the skin-contact opening formed at a lower portion of the lancet assembly 106 and proximate to the bottom portion 102c of the main body 102. In an example, the lancet assembly 106 further includes a release spring 106c, which is configured to retract the needle 106b into the main body 102 after piercing a user's skin.

In an example, the skin-contact opening is formed at a lower region of the lancet assembly 106 and is positioned such that, when the blood extraction device 100 is attached to a user's skin, the needle 106b is configured to pierce the skin through the skin-contact opening, and blood drawn from the skin is directed through the same opening under applied negative pressure.

In an example, the main body 102 of the blood extraction device 100 is formed to include vertical guide walls 118 extending along a longitudinal axis of the blood extraction device 100. The vertical guide walls 118 are configured to receive and guide a needle holder 106b-1 of the lancet assembly 106, such that movement of the needle holder 106b-1 during actuation is constrained to substantially linear axial travel. By mechanically limiting lateral or angular displacement of the needle holder 106b-1, the vertical guide walls 118 ensure accurate advancement and retraction of the needle 106b during actuation, thereby improving penetration consistency, reducing misalignment, and minimizing tissue trauma.

In a further example, the vertical guide walls terminate at a curved recess 108 formed at a lower portion of the main body 102. The curved recess 108 is coaxially aligned with the needle 106b and the needle holder 106b-1 and is positioned directly above a skin-contact opening formed in the main body 102. The curved recess 108 defines a localized collection region configured to receive blood emerging from the user's skin immediately after piercing by the needle 106b. The geometry of the curved recess 108 promotes directional flow of blood toward the skin-contact opening while limiting dispersion or pooling within the device.

In further example, an adhesive layer 110 is disposed on a lower surface of the main body 102, for example on the bottom portion 102c, and is configured to attach the main body 102 to a region of the user's skin such that the region is sealed around the skin-contact opening. The adhesive layer 110 enables formation of a substantially air-tight seal between the blood extraction device 100 and the skin, thereby facilitating effective application of negative pressure from the vacuum chamber 104 to the puncture site.

In an example, a collection chamber 116 is positioned in fluid communication with the skin-contact opening and is configured to receive capillary blood drawn from the user's skin under negative pressure generated by the vacuum chamber 104. In an example, the collection chamber 116 may be removably coupled to the main body 102 and may include anticoagulant material, as described in further detail herein.

By virtue of the disclosed configuration, the blood extraction device provides several technical advantages over conventional blood collection approaches. The mechanical coupling of lancet actuation and vacuum application through a single user action enables immediate application of negative pressure at the puncture site, thereby promoting consistent capillary blood flow without requiring manual squeezing or repeated punctures. The adhesive-based attachment to the skin establishes a sealed interface around the skin-contact opening, which maintains vacuum integrity and minimizes exposure of blood to ambient air, thereby reducing contamination risk and clot formation. Further, automatic retraction of the needle after piercing enhances user safety and reduces discomfort. The integrated blood collection arrangement allows direct transfer of blood into a collection chamber. Collectively, these features enable reliable extraction of a predetermined volume of capillary blood with reduced pain, minimal user intervention, and improved repeatability.

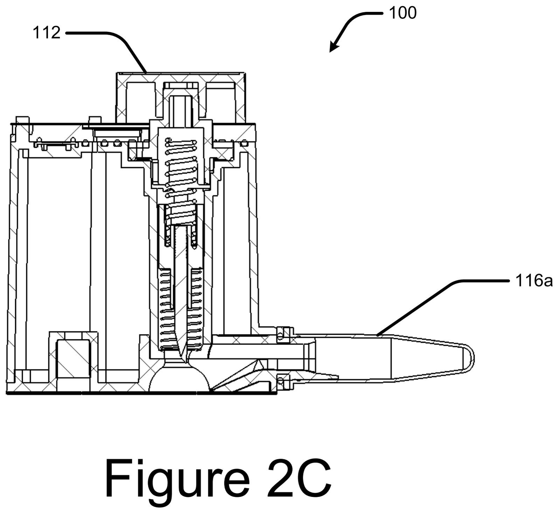

Figure 2C illustrates a cross-sectional view of the blood extraction device 100 , in accordance with another example implementation of the present subject matter. In this example implementation, the overall structural configuration and operational principles of the blood extraction device 100 remain substantially similar to those described with reference to Figures 2A-2B, except that the blood collection arrangement is implemented in the form of a removably attachable collection tube 116a, as described herein.

In an example, the blood extraction device 100 comprises a collection tube 116a that is removably attached to the main body 102. The collection tube 116 is positioned on the side of the blood extraction device 100 such that, when attached, it is in fluid communication with the skin-contact opening through one or more internal flow passages formed within the main body 102. During operation, capillary blood drawn from the user's skin under negative pressure is directed through the skin-contact opening and into the collection tube 116a.

In an example, the collection tube 116a may be preloaded with an anticoagulant, which is configured to prevent or inhibit clotting of blood received within the collection tube 116a during and after collection. This configuration ensures preservation of sample integrity, particularly when handling small volumes of capillary blood intended for downstream diagnostic analysis.

In an example, the collection tube 116a may include a vent (not shown) configured to allow air displaced by incoming blood to exit the collection tube 116a during filling. The vent facilitates smooth and continuous blood inflow by preventing pressure buildup within the collection tube 116a, thereby enabling consistent and controlled collection of capillary blood under applied negative pressure.

In an example, the collection tube 116a is configured to collect a predetermined volume of capillary blood, for example in a range of approximately 100 µL to 200 µL, although other volumes may be accommodated depending on design requirements. Upon completion of blood collection, the collection tube 116a may be detached from the main body 102, capped, and processed independently, while the remaining portions of the blood extraction device 100 may be safely disposed of.

It is to be understood that the use of a removably attachable collection tube 116a as illustrated in Figure 2C represents an alternative implementation of the blood collection arrangement and is provided by way of example only. Such a configuration may be adopted without deviating from the scope of the present subject matter, and other equivalent blood collection arrangements capable of receiving capillary blood under negative pressure may be employed in accordance with the disclosed principles.

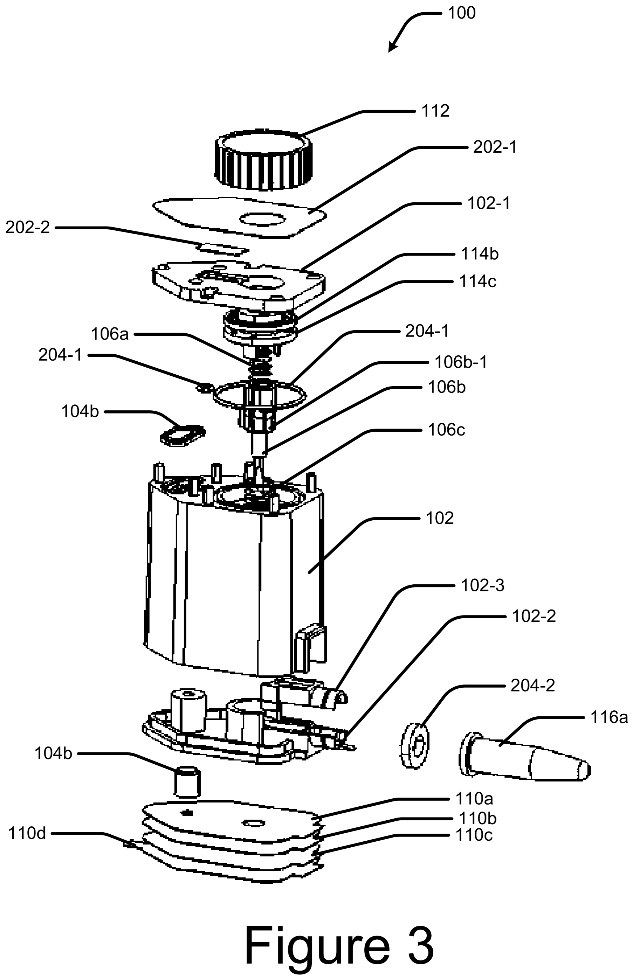

Figure 3 illustrates an exploded view of the blood extraction device 100, in accordance with an example implementation of the present subject matter.

As described herein, the blood extraction device 100 comprises a main body 102 that defines an internal cavity 102a. The internal cavity 102a is configured to receive and support a lancet assembly 106, a valve arrangement, vacuum-related components, fluid communication paths, and interfaces for blood collection. The main body 102 forms the primary structural housing of the blood extraction device 100 and provides mechanical rigidity, positional alignment, and protection for internal components during storage, handling, and use. The main body 102 is further configured to be secured to a user's skin during operation.

The blood extraction device 100 further comprise a base 102-2 is coupled to a lower region of the main body 102 and defines a bottom structural portion of the blood extraction device 100. The base 102-2 cooperates with the main body 102 to enclose the internal cavity 102a and to support vacuum-related sealing components. In some examples, a support part 102-3 is disposed within the internal cavity 102a and is configured to provide mechanical alignment and stabilization for internal assemblies, including the lancet assembly 106 and collection tube 116a. Structural details of the base 102-2 and the support part 102-3 are described in subsequent sections.

The blood extraction device 100 includes a lancet assembly 106 disposed within the internal cavity 102a of the main body 102. The lancet assembly 106 comprises a lancet spring 106a configured to store mechanical energy in a loaded condition prior to actuation, a needle holder 106b-1 configured to guide axial motion, and a needle 106b coupled to the lancet spring 106a and aligned along a longitudinal axis of the device. The lancet assembly 106 further includes a release spring 106c, which is configured to retract the needle 106b back into the main body 102 after skin penetration. This automatic retraction limits needle exposure after use and enhances user safety.

To selectively control application of negative pressure, the blood extraction device 100 includes a valve arrangement comprising a rubber valve 114b mounted on a valve base 114c. The rubber valve 114b is movable between a closed configuration, in which fluid communication is blocked, and an open configuration, in which fluid communication is established. In an example, a large O-ring 204-1 may be positioned around the valve arrangement to provide an air-tight seal between the rubber valve 114b, the valve base 114c, and adjacent surfaces of the main body 102, thereby preserving vacuum integrity prior to actuation and preventing leakage during valve movement.

The valve arrangement of the blood extraction device 100 is operatively coupled to an actuator knob 112, which serves as a user-accessible actuation interface. In an example, the actuator knob 112 may be disposed on an upper surface 102b of the main body 102 formed by a top cover 102-1. The top cover 102-1 forms the top portion 102b of the main body 102 and provides structural support and proper positional alignment between the actuator knob 112, the valve arrangement, and the lancet assembly 106. Actuation of the actuator knob 112, for example by rotation, causes coordinated movement of the rubber valve 114b and initiates operation of the lancet assembly 106.

In some examples, a top label sticker 202-1 may be disposed on an exterior surface of the top cover 102-1 and is configured to cover, seal, or protect one or more internal openings or conduits formed in the top cover 102-1. In an example, a top acrylic plate channel sticker 202-2 may be positioned on the top cover 102-1 and bonded to an underlying plate. The top acrylic plate channel sticker 202-2 defines one or more channel features that cooperate with adjacent surfaces of the main body 102 to form a vacuum-sealed flow path in fluid communication between a vacuum chamber 104 and a needle or skin-contact region of the blood extraction device 100.

The blood extraction device 100 further includes a vacuum chamber 104 (not shown in Figure 3) defining a sealed internal volume configured to contain a prefilled vacuum. In one example, a vacuum seal rubber 104a may be provided at a bottom portion of the blood extraction device 100 and is accessible from an underside of the blood extraction device 100. The vacuum seal rubber 104a functions as a sealing interface that maintains the prefilled vacuum within the vacuum chamber 104 after manufacturing. Additionally, in some examples, the vacuum seal rubber 104a permits recreation or restoration of vacuum within the vacuum chamber 104, for example in the event of leakage during storage or handling, without requiring disassembly of the device.

In some examples, the blood extraction device 100 may include a vacuum indicator 104b configured to provide a visual indication of the presence or absence of sufficient negative pressure within the vacuum chamber 104. In one implementation, the vacuum indicator 104b comprises a mechanically responsive diaphragm that deforms in response to pressure conditions within the device. The diaphragm may be exposed at an external surface of the main body 102 such that a change in shape is visible to a user. For example, the diaphragm may assume a concave configuration when negative pressure is present and a substantially flat configuration when negative pressure is absent. In an example, the vacuum indicator 104b may be disposed on a top surface 102b or a side surface of the main body 102 to enable verification of device readiness prior to actuation.

The blood extraction device 100 further includes a collection tube 116a configured to be removably attached to the main body 102. When attached, the collection tube 116a is positioned in fluid communication with a skin-contact opening of the device and is configured to receive capillary blood drawn under negative pressure from the vacuum chamber 104. A silicon O-ring 204-2 may be disposed at an interface between the collection tube 116a and the main body 102 to provide a fluid-tight and air-tight seal, thereby preventing leakage and maintaining vacuum integrity during blood collection.

The lower surface 102c of the blood extraction device 100, which is a skin-facing side of the device, comprises a multilayer adhesive assembly to attach the blood extraction device 100 to a user's skin. In an example, the adhesive assembly comprises a double-sided sticker 110a disposed on the lower portion of the main body 102, a PC sheet 110b positioned below the double-sided sticker 110a to provide structural support, a skin sticker 110c disposed below the sheet and configured to adhere directly to the user's skin, and a peel-off sticker 110d removably coupled to the skin sticker 110c and configured to be removed prior to use. These layers collectively establish a substantially leak-proof seal around a skin-contact region, which is critical for maintaining vacuum pressure during blood extraction.

In an assembled state, the blood extraction device 100, a single actuation of the actuator knob 112, which is rotatably supported by the top cover 102-1 forming the top portion 102b of the main body 102, initiates coordinated mechanical and fluidic operations within the device. The actuator knob 112 is operatively coupled to a rubber valve 114b mounted on a valve base 114c, such that rotation of the actuator knob 112 causes corresponding rotational displacement of the rubber valve 114b relative to the valve base 114c. A large O-ring 204-1 may be disposed between the rubber valve 114b, the valve base 114c, and adjacent surfaces of the main body 102 to maintain an air-tight seal during rotation and to prevent unintended vacuum leakage. In an initial, pre-actuation position, the rubber valve 114b blocks fluid communication between a vacuum chamber 104, which contains a prefilled vacuum retained by a vacuum seal rubber 104a, and a downstream flow path leading to a skin-contact opening. Upon actuation, rotation of the actuator knob 112 repositions the rubber valve 114b to align internal openings with channel features defined by a top acrylic plate channel sticker 202-2 and adjacent surfaces of the main body 102, thereby establishing fluid communication between the vacuum chamber 104 and the skin-contact opening.

Substantially simultaneously, the actuator knob 112 mechanically releases a lancet assembly 106 disposed within the internal cavity of the main body 102. The lancet assembly 106 includes a lancet spring 106a maintained in a compressed, loaded state, a needle holder 106b-1 guided for linear movement, and a needle 106b coupled to the lancet spring 106a. Release of the lancet spring 106a drives the needle holder 106b-1 having the needle 106b downward toward the skin-contact opening, causing the needle 106b to pierce the user's skin. Immediately after piercing, a release spring 106c retracts the needle holder 106b-1 and needle 106b back into the main body 102, thereby limiting needle exposure. As the rubber valve 114b simultaneously places the vacuum chamber 104 in fluid communication with the skin-contact opening, negative pressure is applied to the puncture site, causing capillary blood to be drawn through the opening and into downstream blood collection components. Accordingly, the mechanical coupling between the actuator knob 112, the rubber valve 114b, and the lancet assembly 106 ensures that needle piercing and vacuum application occur in a synchronized manner through a single user actuation, thereby improving reliability, reducing user intervention, and enabling consistent capillary blood extraction.

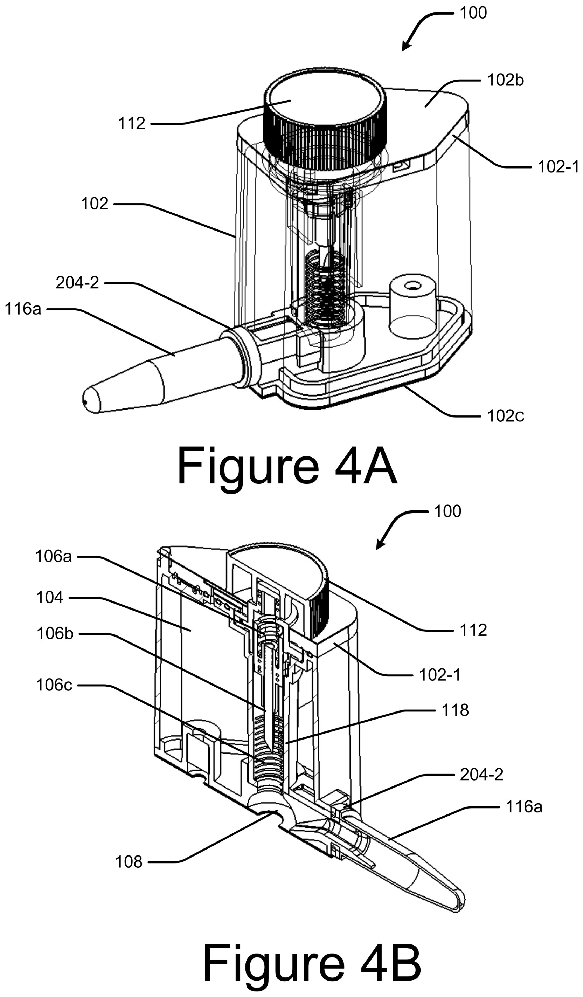

Figures 4A and 4B respectively illustrate a perspective sectional view and a cross-sectional view of the blood extraction device 100 in a loaded state, in accordance with an example implementation of the present subject matter. As used herein, the loaded state refers to a configuration in which the lancet assembly 106 is maintained in a pre-actuation condition with the lancet spring 106a held under stored energy and the vacuum chamber 104 retaining prefilled negative pressure, prior to user actuation.

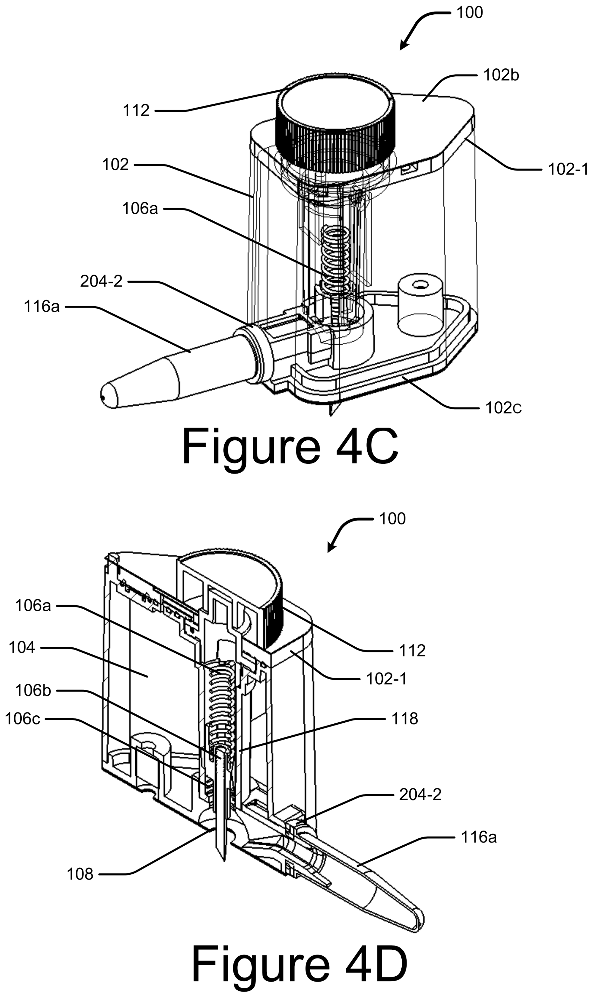

Figure 4C illustrates another perspective sectional view and Figure 4D illustrates another cross-sectional view of the blood extraction device 100 in an unloaded state, in accordance with an example implementation of the present subject matter. As used herein, the unloaded state refers to a configuration in which the lancet assembly has been actuated and the stored energy of the lancet spring has been released, with the needle 106b retracted and the vacuum having been applied to the skin-contact region during blood extraction.

For the sake of clarity and brevity, Figures 4A-4D are described together herein, as they collectively depict the structural and functional differences between the loaded state and the unloaded state of the blood extraction device.

In use, the blood extraction device 100 may be initially provided in a packaged condition with the lancet assembly maintained in a loaded state and a vacuum chamber retaining prefilled negative pressure, as illustrated in Figures 4A and 4B. Prior to use, a user removes the blood extraction device 100 from its packaging and removes a peel-off protective layer 110d covering a skin-contact adhesive provided on a bottom portion of the device. The blood extraction device 100 is then positioned against a target skin region of the user, for example on an upper arm, such that the bottom portion of the blood extraction device 100 faces the skin. The blood extraction device 100 is oriented such that a collection tube 116a coupled to the blood extraction device 100 is faced downwards towards the ground. This orientation allows gravity to assist in directing extracted capillary blood downward into the collection tube 116a during operation, thereby improving collection efficiency and reducing the likelihood of blood pooling within the device.

Once positioned, the user presses the blood extraction device 100 against the skin, causing the skin sticker 110c of the adhesive layer 110 to bond to the skin and form a substantially leak-proof seal around the skin-contact opening. At this stage, as shown in Figures 4A and 4B, the blood extraction device 100 remains in the loaded state, wherein the lancet spring 106a is held in a compressed condition, the needle 106b is retained in a retracted position, and the vacuum chamber 104 remains isolated from the skin-contact opening.

The user then actuates the blood extraction device 100 by rotating an actuator 112 provided on a top surface 102b of the blood extraction device 100. The rotation of the actuator 112 initiates coordinated internal actions. First, stored mechanical energy in the lancet assembly is released, driving a needle holder 106b-1 and an associated needle 106b toward the skin for penetration. The needle holder is guided within vertical guide walls 118 formed in the main body 102, which constrain the needle holder 106b-1 to linear axial movement and prevent lateral deflection during actuation. This guided motion ensures accurate penetration of the skin.

As the needle holder advances, the needle 106b pierces the skin through the skin-contact opening to create a controlled capillary puncture. The vertical guide walls 118 extend downward and terminate in a curved recess 108 formed at a lower portion of the main body 102. The curved recess 108 may be coaxially aligned with the needle and positioned above the skin-contact opening. This geometry directs blood emerging from the puncture site toward the skin-contact opening while minimizing dispersion and loss. Immediately after skin penetration, a release spring 106c associated with the lancet assembly 106 retracts the needle 106b and needle holder 106b-1 back into the main body 102, returning the lancet assembly 106 to a retracted condition. This transition marks the beginning of the unloaded state, as illustrated in Figures 4C and 4D, and ensures that the needle is no longer exposed following puncture.

Substantially simultaneously with release of the lancet assembly, continued rotation of the actuator 112 causes a valve arrangement within the blood extraction device 100 to change configuration, thereby placing the vacuum chamber 104 in fluid communication with the skin-contact opening. The resulting pressure differential applies negative pressure at the puncture site. Capillary blood is drawn from the skin, guided by the curved recess 108 and skin-contact opening, and directed into the collection tube 116a.

As blood flows into the collection tube 116a, air displaced by the incoming blood exits through a vent, enabling uninterrupted flow. The downward orientation of the collection tube allows gravity to assist in blood accumulation within the tube. Over a predefined collection interval, capillary blood accumulates within the collection tube, which may contain an anticoagulant to inhibit clot formation and preserve sample integrity.

Upon completion of blood collection, the blood extraction device 100 remains in the unloaded state, with the lancet assembly fully retracted and the vacuum having been applied. The device may then be removed from the skin, the collection tube detached and sealed for testing, and the remaining portions of the device safely disposed of. The adhesive seal is released from the skin, and the puncture site may be cleaned and dressed as appropriate.

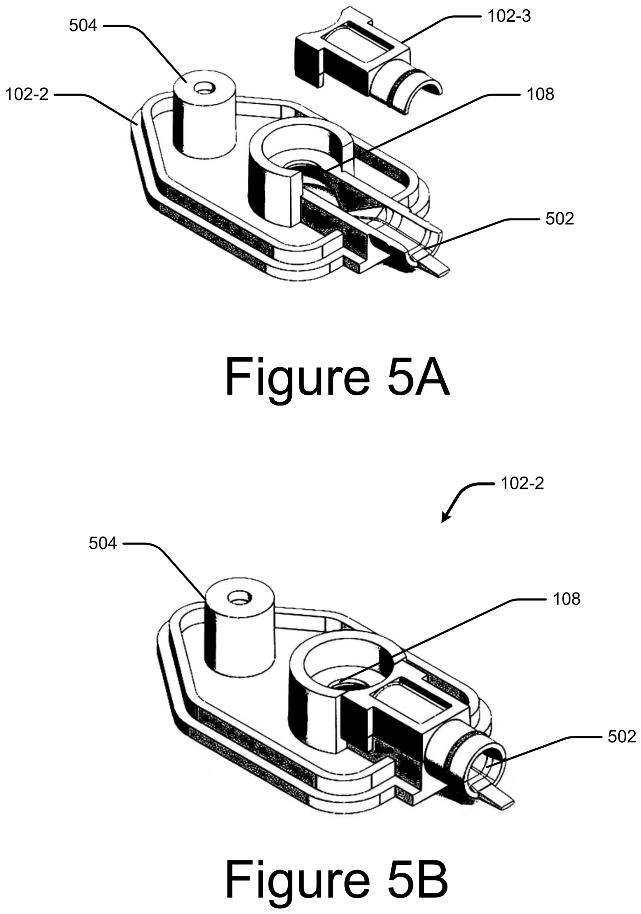

Figure 5A-5B illustrates perspective views of a base 102-2 of the blood extraction device 100 and an associated support part 102-3, in accordance with an example implementation of the present subject matter. The illustrated views show the base 102-2 and the support part 102-3 in a separated state and in an assembled state, respectively, to highlight their structural cooperation and functional roles.

In an example, the base 102-2 defines a lower structural component of the blood extraction device 100 and forms the lower portion of the main body 102. The base 102-2 provides mechanical support, defines fluid routing features, and houses vacuum-related sealing elements. The base 102-2 further forms part of a skin-facing region of the device and cooperates with adhesive layers described elsewhere herein.

In the illustrated example, the base 102-2 includes an opening 502, which is configured to receive and retain a collection tube (for example, collection tube 116a, not shown in this figure). The opening 502 is in fluid connection with the curved recess 108 and defines a fluid inlet region through which capillary blood drawn from a skin-contact region enters the collection tube during operation. The opening 502 is positioned and oriented such that, when the device is attached to a user's skin, the collection tube extends downwardly from the base 102-2, thereby allowing gravity to assist in directing blood into the collection tube.

The base 102-2 further includes a curved recess 108, which is formed at an upper region of the base 102-2 and is positioned to be coaxially aligned with the needle and skin-contact opening of the device when assembled. The recess 108 is configured to receive blood emerging from a puncture site immediately after skin penetration and to guide the blood toward the opening 502. The geometry of the recess 108 promotes controlled flow toward the collection tube under applied negative pressure.

Additionally, the base 102-2 includes a housing 504 formed as a cylindrical or cavity-like structure configured to accommodate a vacuum seal rubber 104a (not shown in this figure). The housing 504 defines a sealed interface that cooperates with the vacuum seal rubber 104a to create and retain a vacuum within an associated vacuum chamber. The housing 504 is accessible from the underside of the base 102-2, thereby enabling sealing of a prefilled vacuum during manufacturing and, in some examples, allowing restoration of vacuum in the event of leakage.

The support part 102-3 is illustrated as a separate component configured to be mounted within or adjacent to the base 102-2. The support part 102-3 is shaped to engage corresponding features of the base 102-2 and provides mechanical alignment, positional support, and sealing cooperation for internal components, including portions of a lancet assembly, fluid passages, or vacuum-related interfaces. When assembled, the support part 102-3 cooperates with the base 102-2 to define internal cavities and flow paths while maintaining structural rigidity.

As shown in the assembled view of Figure 5B, the support part 102-3 is received within the base 102-2 such that the opening 502, recess 108, and housing 504 are maintained in fixed spatial alignment relative to one another. This arrangement ensures reliable fluid communication between the skin-contact region and the collection tube.

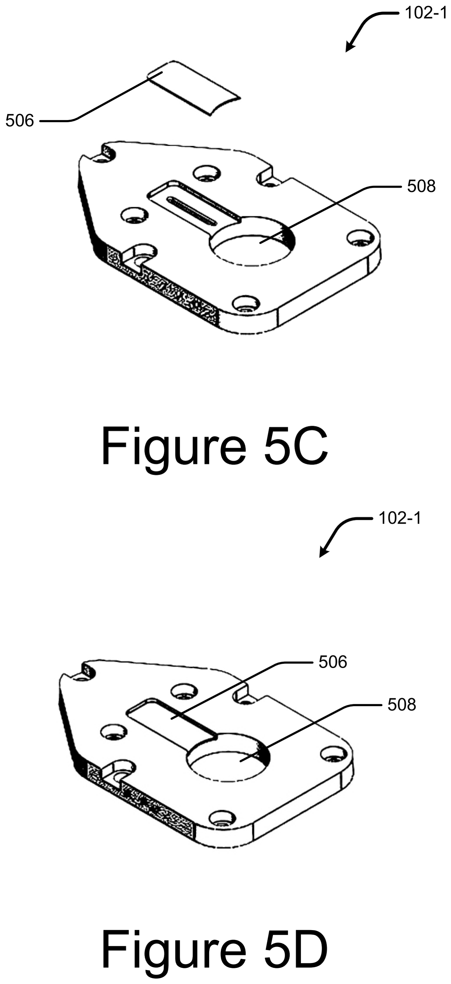

Figures 5C and 5D illustrate perspective views of a top plate 102-1 of the blood extraction device 100, in accordance with an example implementation of the present subject matter. Figures 5C and 5D are described together herein for clarity, as they depict the same top plate in different assembly states.

As illustrated in Figure 5C, the top plate 102-1 defines an upper structural component of the blood extraction device 100 and includes one or more openings, recesses, and surface features configured to cooperate with internal components of the device. In the illustrated example, the top plate 102-1 includes a channel layer 508, shown separately from the top plate, prior to assembly.

The channel layer 508 is configured to be arranged on and bonded to a surface of the top plate 102-1. The channel layer 508 defines one or more channel features that, when bonded to the top plate 102-1, form a vacuum-sealed flow path. This flow path is configured to provide fluid communication between a vacuum chamber of the blood extraction device and a needle area associated with a lancet assembly.

Figure 5D illustrates the top plate 102-1 after the channel layer 508 has been positioned and bonded thereto. In the assembled configuration shown in Figure 5D, the channel layer 508 cooperates with adjacent surfaces of the top plate 102-1 to define a sealed internal passage, thereby enabling controlled transmission of negative pressure from the vacuum chamber toward the needle area during device operation.

As further shown in Figures 5C and 5D, the region indicated by reference numeral 508 also corresponds to a mounting and alignment location for the lancet assembly. In particular, the channel layer 508 is positioned such that, when the lancet assembly is installed, the needle is aligned with the vacuum-sealed flow path formed by the channel layer 508 and the top plate 102-1, thereby enabling application of negative pressure at a skin-contact opening immediately after skin penetration.

Accordingly, Figures 5C and 5D illustrate a structural arrangement in which the top plate 102-1 and the bonded channel layer 508 collectively define a sealed fluidic interface between the vacuum chamber and the needle area, while also providing positional alignment for the lancet assembly.

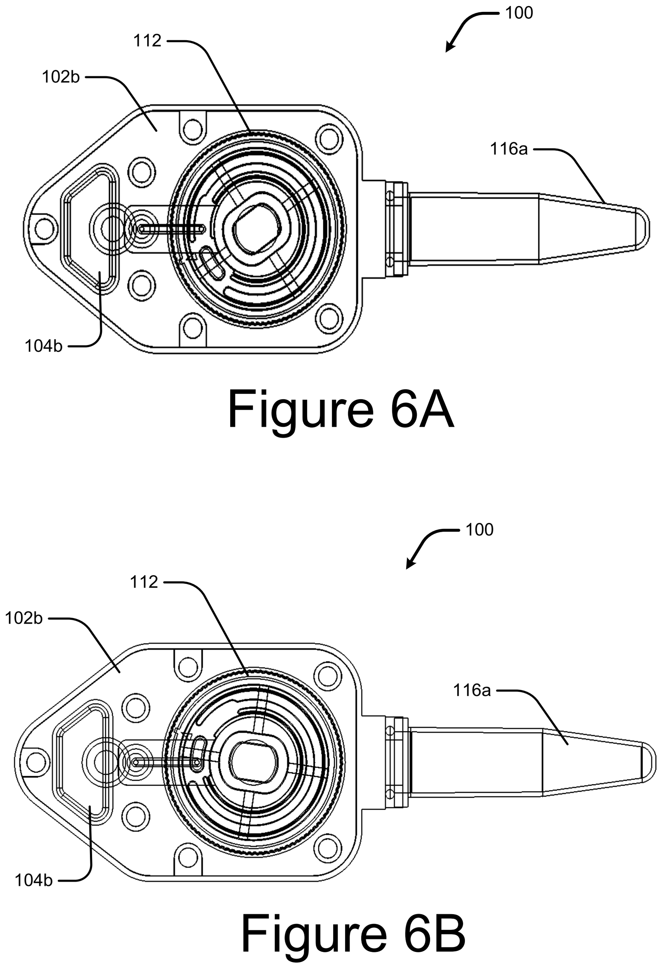

Figures 6A and Figure 6B illustrate top views of the blood extraction device 100 in different operational states and are explained together herein for the sake of brevity. In particular, Figure 6A depicts the blood extraction device 100 in a loaded state, while Figure 6B depicts the blood extraction device 100 in an unloaded state, with each figure showing a corresponding position of an actuator 112.

The blood extraction device 100 comprises a main body having a top portion 102b, within which the actuator 112 is disposed. The actuator 112 is accessible from an upper surface of the top portion 102b and is configured to be manually actuated by a user, for example by rotation, to initiate operation of the blood extraction device 100, as described herein.

In the loaded state, as illustrated in Figure 6A, the actuator 112 is positioned at an initial angular or rotational position relative to the top portion 102b of the main body. In this state, internal components of the blood extraction device 100, including a lancet assembly and a vacuum arrangement, are maintained in a pre-actuation condition. Specifically, the lancet assembly remains restrained in a loaded configuration, and a valve arrangement remains positioned such that a vacuum chamber is isolated from a skin-contact region. The position of the actuator 112 in Figure 6A visually indicates that the blood extraction device 100 is ready for use but has not yet been actuated.

In contrast, Figure 6B illustrates the blood extraction device 100 in the unloaded state, following actuation by the user. In this state, the actuator 112 has been moved, for example rotated, from the initial position shown in Figure 6A to a second position shown in Figure 6B. Movement of the actuator 112 corresponds to internal operation of the device, including release of the lancet assembly and reconfiguration of the valve arrangement to apply vacuum at a skin-contact opening. The position of the actuator 112 in Figure 6B thus provides a visual indication that the blood extraction device 100 has been actuated.

As further illustrated in Figures 6A and 6B, a collection tube 116a is coupled to the blood extraction device 100 and extends laterally from the main body. The collection tube 116a is positioned such that, during use, it may be oriented below the skin-contact opening to facilitate gravity-assisted collection of capillary blood under applied negative pressure. The collection tube 116a remains coupled to the blood extraction device 100 in both the loaded and unloaded states.

Accordingly, Figures 6A and 6B collectively illustrate how the position of the actuator 112 on the top portion 102b of the blood extraction device 100 changes between the loaded and unloaded states, thereby providing a clear visual representation of device readiness and actuation status while maintaining the same overall external configuration of the device.

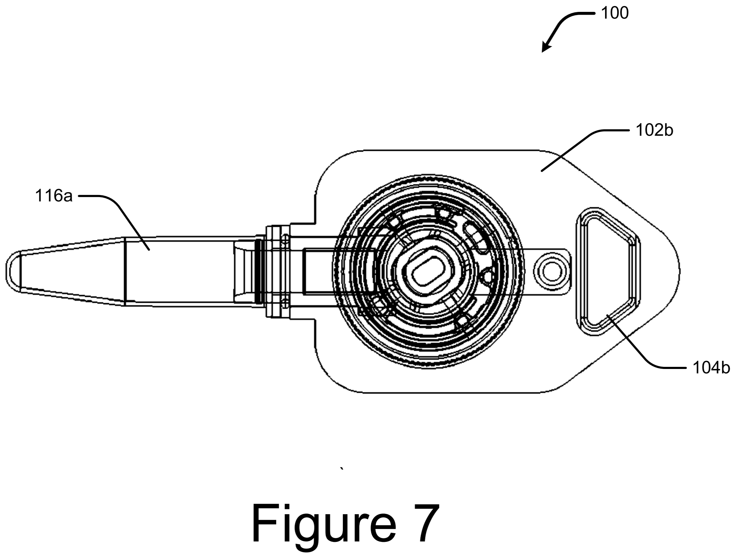

Figure 7 illustrates the top view of the blood extraction device having a vacuum indicator, in accordance with another example of a present subject matter;

As illustrated, the blood extraction device 100 comprises a main body having a top portion 102b, on which an actuation interface and internal components are housed. The top portion 102b provides a planar upper surface through which the internal configuration of the device may be visually inferred and on which user-visible indicators are positioned.

In an example, the vacuum indicator 104b is disposed on the top portion 102b of the main body. The vacuum indicator 104b is configured to provide a visual indication of a vacuum status within the blood extraction device 100. In one example, the vacuum indicator 104b comprises a diaphragm or deformable element that changes shape in response to pressure conditions within a vacuum chamber of the device. For example, the vacuum indicator 104b may exhibit a concave appearance when sufficient negative pressure is present and a substantially flat appearance when negative pressure is absent or reduced.

The vacuum indicator 104b is positioned such that it is readily visible to a user from the top view, thereby enabling verification of device readiness prior to actuation and confirmation of vacuum release after use. Placement of the vacuum indicator 104b on the top portion 102b allows the user to assess vacuum status without rotating or repositioning the blood extraction device 100.

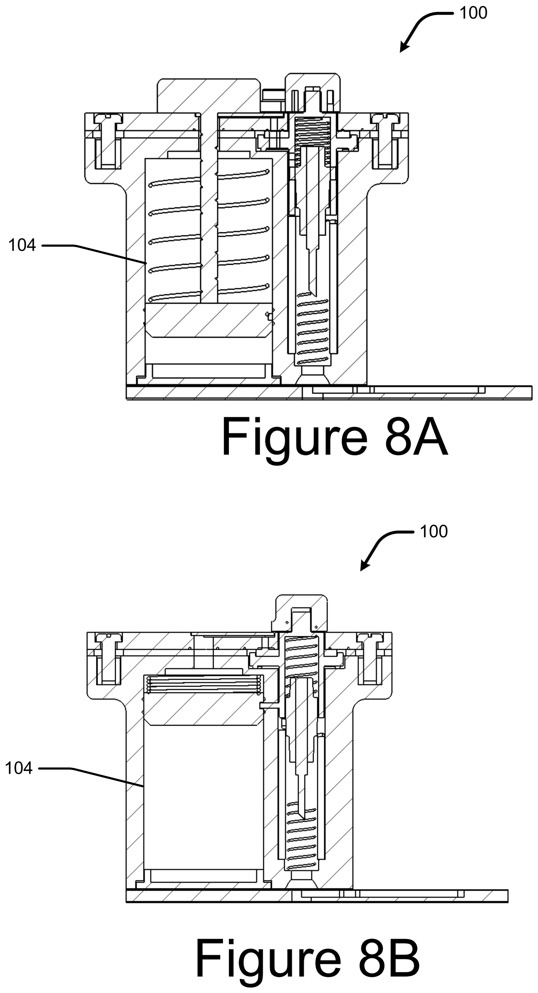

Figure 8A illustrates a front view of the blood extraction device 100, in accordance with another example of a present subject matter.

In the example illustrated in Figure 8A, the blood extraction device 100 employs an alternative vacuum generation arrangement as compared to embodiments described previously. In this embodiment, the blood extraction device 100 includes a spring and piston assembly operatively coupled to a user-actuatable knob for generating negative pressure. Unlike embodiments in which the vacuum chamber 104 contains a prefilled vacuum, the vacuum chamber 104 in the present embodiment is initially at or near atmospheric pressure, and vacuum is created by user actuation prior to lancet actuation.

In operation, rotation or actuation of the knob causes movement of the piston against the spring, thereby evacuating the vacuum chamber 104 and generating negative pressure within the chamber. Once the desired level of negative pressure is established, the vacuum chamber 104 is configured to retain the generated vacuum until subsequent actuation of the device to initiate blood extraction.

In the embodiment illustrated in Figure 8A, following vacuum generation, the actuator knob is further operable to initiate a coordinated sequence of actions. Upon single actuation of the actuator, movement of the actuator simultaneously drives the piston against the spring to generate negative pressure within the vacuum chamber 104, and also initiates operation of the lancet assembly to pierce the skin. The generated negative pressure is placed in fluid communication with the skin-contact opening during the same actuation cycle, thereby enabling coordinated skin piercing and vacuum-assisted blood extraction through a single user input.

. In this embodiment, negative pressure required for capillary blood extraction is generated prior to actuation of the lancet assembly by operation of a spring-and-piston arrangement, rather than by a prefilled vacuum, while maintaining compatibility with the overall configuration and operation of the blood extraction device 100.

Figure 8B illustrates a front view of the blood extraction device, in accordance with another example of a present subject matter;

In the example illustrated in Figure 8B, the blood extraction device 100 includes another alternative vacuum generation arrangement comprising a spring-loaded piston retained in a compressed state by a release mechanism. In this embodiment, the vacuum chamber 104 does not contain a prefilled vacuum and is initially at or near atmospheric pressure prior to use.

In operation, the spring-loaded piston is maintained in a restrained position by the release mechanism until actuated by the user. Upon single actuation of the actuator, the release mechanism is disengaged, allowing the spring-loaded piston to move and generate negative pressure within the vacuum chamber 104, while simultaneously releasing the lancet assembly to perform skin puncture. During the same actuation, the valve arrangement places the vacuum chamber 104 in fluid communication with the skin-contact opening, such that vacuum application and needle piercing occur in a synchronized manner.

In the embodiment illustrated in Figure 8B, the mechanism for generating negative pressure differs from that described with reference to earlier embodiments. Except for this difference, the structural arrangement, component configuration, and functional cooperation of the remaining elements of the blood extraction device 100 are substantially similar to those previously described and are therefore not repeated herein. In this embodiment, negative pressure required for capillary blood extraction is generated by release of a spring-loaded piston prior to initiation of blood extraction, rather than by a prefilled vacuum or manual piston actuation, while maintaining compatibility with the overall configuration and operation of the blood extraction device 100.

In an example, manufacture of the blood extraction device 100 includes forming a top plate and channel assembly, wherein a channel layer 202-2 is arranged on and bonded to a top plate 102-1. The channel layer 202-2 cooperates with the top plate 102-1 to define a vacuum-sealed flow path configured to establish fluid communication between a vacuum chamber 104 and a needle area associated with the lancet assembly 106. The bonding may be achieved using adhesive bonding, thermal bonding, or equivalent permanent joining techniques to ensure vacuum integrity of the formed flow path.

In a further step, a base plate 102-2 and a support plate 102-3 are assembled. The support plate 102-3 is positioned relative to the base plate 102-2 and secured thereto using a permanent bonding process, such as heat staking, ultrasonic welding, or adhesive bonding. This assembly defines structural support features and alignment interfaces for downstream components, including a collection tube interface 502 and vacuum-related sealing elements, including a housing 504 configured to receive a vacuum seal rubber 104a.

In another step, a needle assembly is formed by assembling a needle 106b with a needle holder 106b-1 to create an integrated subassembly. The needle assembly is configured to be later positioned within a main body 102 of the blood extraction device 100. Subsequently, the base plate 102-2 is bonded to the main body 102, thereby forming a vacuum-tight sealed structure in which the vacuum chamber 104 is defined within the main body 102. In some examples, a pre-vacuum is created and retained within the vacuum chamber 104 at this stage.

Thereafter, the top plate 102-1 is assembled with the main body 102, during which internal components including one or more O-rings 204-1, 204-2, a rotary valve base 114c, a rotary rubber valve 114b, a vacuum-indicating rubber 104b, one or more springs 106a, 106c, and the needle holder 106b-1 are arranged within the main body 102 using a fixture. The fixture is closed and a heat-staking operation is performed to secure the assembly. In a final stage, a silicone rubber component 104a and a collection tube 116a are attached, followed by application of an adhesive assembly 110a-110d, an actuator knob 112, and external stickers 202-1. In an example, the channel region may be treated with hydrophilic and anticoagulant agents, after which a vacuum is applied to the vacuum chamber 104 and the blood extraction device 100 is sealed to retain the vacuum.

The present invention offers a faster, one-step process for blood extraction, making it ideal for at-home testing, self-diagnostic kits, and point-of-care devices, where simplicity, speed, and ease of use are essential.

Although examples for the present disclosure have been described in language specific to structural features and/or methods, it is to be understood that Such examples are not necessarily limited to the specific features or methods described. Rather, the specific features and methods are disclosed and explained as examples of the present description.

## Claims

1. A blood extraction device (100) comprising:
a main body (102) defining an internal cavity (102a), the internal cavity (102a) comprising:
a vacuum chamber (104), and
a lancet assembly (106) comprising a lancet spring (106a) and a needle (106b) coupled to the lancet spring (106a), the needle (106b) being aligned with a skin-contact opening formed at a lower surface of the main body (102);
an adhesive layer (110) disposed on the lower surface of the main body (102) and configured to attach the main body (102) to a region of a user's skin such that the region is sealed around the skin-contact opening;
an actuator knob (112) mounted on a top surface (102b) of the main body (102);
a rotating valve (114b) positioned within the internal cavity (102a) and coupled to the lancet assembly (106), the rotating valve (114b) comprising a valve port configured to align with a fluid passage extending between the vacuum chamber (104) and the skin-contact opening in the main body (102);
wherein the actuator knob (112) is mechanically coupled to the rotating valve (114b) and to the lancet spring (106a) such that rotation of the actuator knob (112) actuates the lancet spring (106a) to move the needle (106b) toward the skin-contact opening to pierce the user's skin and simultaneously rotates the rotating valve (114b) to align the valve port with the fluid passage causing negative pressure of the vacuum chamber (104) to be in fluid alignment with the skin-contact opening;
wherein blood is drawn from the user's skin through the skin-contact opening under the negative pressure.

2. The blood extraction device (100) as claimed in claim 1, further comprising a blood collection tube (116a) removably attached to the main body (102) and in fluid communication with the skin-contact opening, the blood collection tube (116a) being configured to receive capillary blood drawn under negative pressure from the vacuum chamber (104).

3. The blood extraction device (100) as claimed in claim 2, wherein the blood collection tube (116a) contains an anticoagulant configured to prevent clotting of blood received through the skin-contact opening.

4. The blood extraction device (100) as claimed in claim 2 or claim 3, wherein the blood collection tube (116a) comprises a vent configured to allow air displaced during blood inflow to exit the blood collection tube (116a).

5. The blood extraction device (100) as claimed in any one of claims 2 to 4, wherein the blood collection tube (116a) is configured to collect a volume of capillary blood in a range of 100 µL to 200 µL.

6. The blood extraction device (100) as claimed in any one of claims 1 to 5, wherein the adhesive layer (110) comprises a multilayer laminate including:
a double-sided adhesive sticker (110a) disposed on the lower surface of the main body (102);
a sheet (110b) positioned below the double-sided adhesive sticker (110a) to provide structural support;
a skin sticker (110c) disposed below the sheet and configured to adhere to the user's skin to maintain a leak-proof seal around the skin-contact opening; and
a peel-off sticker (110d) removably coupled to the skin sticker and configured to be removed prior to attachment to the user's skin.

7. The blood extraction device (100) as claimed in any one of claims 1 to 6, wherein the lancet assembly (106) further comprises a release spring (106c) positioned to retract the needle (106b) into the main body (102) after piercing the user's skin.

8. The blood extraction device (100) as claimed in any one of claims 1 to 7, further comprising a vacuum indicator (104b) comprising a diaphragm configured to assume a concave shape when the vacuum chamber (104) contains negative pressure and a substantially flat shape when the vacuum chamber (104) does not contain negative pressure.

9. The blood extraction device (100) as claimed in claim 8, wherein the vacuum indicator (104b) is disposed on a surface of the main body (102) and includes the diaphragm exposed at an external surface of the main body (102).

10. The blood extraction device (100) as claimed in claim 9, wherein the vacuum indicator (104b) is disposed on the top surface (102b) of the main body (102) or on a side surface of the main body (102).

11. The blood extraction device (100) as claimed in any one of claims 1 to 10, wherein a needle holder (106b-1) is guided within vertical guide walls (118) formed in the main body (102) to ensure linear needle travel during actuation.

12. The blood extraction device (100) as claimed in claim 11, wherein the vertical guide walls (118) end with a curved recess (108) formed at a lower portion of the main body (102), the curved recess (108) being coaxially aligned with the needle (106b) and positioned above the skin-contact opening.

13. The blood extraction device (100) as claimed in claim 12, wherein the curved recess (108) is configured to receive blood emerging from the user's skin after piercing by the needle (106b), the curved recess (108) having an outlet opening (502) configured to establish fluid communication with the blood collection tube (116a) to collect the blood.
